# EUROPEAN PATENT APPLICATION

(11) **EP 4 249 032 A1**
(43) Date of publication of application: **27.09.2023**
(21) Application number: 22163736.6
(22) Date of filing: 23.03.2022
(51) Int. Cl.: A61M 25/01, A61B 10/00

(54) **CATHETER, CATHETER HANDLE**

(71) Applicant: Settergren, Magnus, 113 33 Stockholm (SE); Holmin, Staffan, 16754 Bromma (SE)
(72) Inventor: SETTERGREN, Magnus, 113 33 Stockholm (SE); HOLMIN, Staffan, 16754 Bromma (SE); HAARSTAD, Phil, Fridley, 55421 (US)
(74) Representative: KIPA AB

(57) **Abstract**

A catheter (1) for delivery and sampling comprises a flexible and cylindrical hollow tube (2) at a distal end, the flexible tube (2) has two opposite ends (3, 4), a first side (5) and a to the first side (5) opposite second side (6) extending between the two opposite ends (3, 4), wherein the first side (5) has cutouts (7) along at least a part of its length, and wherein a pull wire (8) is arranged at the most distal end (3) and on the first side (5) of the flexible tube (2). The second side (6) has cutouts (9) along a corresponding length to the first side (5) and wherein the cutouts (7) on the first side are wider than the cutouts (9) on the second side (6).

## Description

### TECHNICAL FIELD

The present invention relates to a catheter for delivery and sampling that comprises a flexible and cylindrical hollow tube at a distal end. The flexible tube has two opposite ends, a first side and a to the first side opposite second side extending between the two opposite ends. The first side has cutouts along at least a part of its length, and wherein a pull wire is arranged at the most distal end and on the first side of the flexible tube.

The present invention further relates to a catheter handle having a distal end and a proximal end, the handle comprising an elongate housing extending between the proximal end and the distal end. The housing has an opening at the distal end arranged to allow one pull wire for a unidirectional catheter to run through. A holding device is slidably arranged along and inside the housing and arranged to be fixed to the pull wire. The handle further comprises a pull wire control arranged such that it is accessible on the outside of the housing and connected through a side opening in the housing to the holding device.

Further, the present invention further relates to a method of guiding a catheter to an endocardial site, lungs, epicardial site via coronary vessels, or abdominal organs.

### BACKGROUND

Conventionally, heart surgery is an open-heart procedure conducted under general anesthesia, during which the heart is stopped and blood flow is controlled by a heart-lung bypass machine. Traditional open surgery inflects significant patient trauma and discomfort and exposes the patient to a number of potential risks, such as an infection, stroke, renal failure, and adverse effects associated with the use of the heart-lung machine, for example.

Due to the drawbacks of open-heart surgical procedures, there has been an increased interest in minimally invasive procedures. With percutaneous transcatheter (or transluminal) techniques, a catheter can be advanced, for example, through an opening in the femoral artery and through the descending aorta to the heart, where, for instance, a prosthesis is then deployed in the annulus of the valve to be repaired (e.g., the aortic valve annulus).

However, there remains a need for improved methods and systems for further minimizing patient trauma and discomfort after using catheters.

### SUMMARY

It is thus an object of the present invention to provide a system and method that further decreases the issues with minimally invasive procedures.

According to a first aspect of the present disclosure a catheter for delivery and sampling comprises a flexible and cylindrical hollow tube at a distal end is provided. The flexible tube has two opposite ends, a first side and a to the first side opposite second side extending between the two opposite ends. The first side has cutouts along at least a part of its length, and wherein a pull wire is arranged at the most distal end and on the first side of the flexible tube. The second side has cutouts along a corresponding length to the first side and wherein the cutouts on the first side are wider than the cutouts on the second side. Such a catheter provide for the possibility to reach the heart via radial access or the cubital vein. Gaining access to the heart via the cubital vein or radial access lowers the discomfort of a patient as compared not only to open-heart surgery but also to minimally invasive procedure via the femoral artery or the jugular vein. By using said catheter that is unilateral it is possible to provide a small enough dimension of the catheter and thereby to open up the possibility for entering via a radial access or the cubital vein. A specific design with a dilator that runs over a 0,014-inch (0,3556 mm) or a 0,035-inch (0,889 mm) guide wire enables the device to be used without an introducer. A pre-inserted dilator is withdrawn when the aorta or caval veins have been reached.

Further, the present invention also relates to a combined introducer and actively deflectable guide catheter for delivery and sampling that comprises a flexible and cylindrical hollow tube at a distal end according to the above disclosure.

The specific design of the flexible tube that is deflectable in only one direction makes it possible to produce catheters in smaller dimensions as low as about 3 French and still maintain torqueability and sufficient strength.

According to a further aspect of the present disclosure the cutouts on the first side are V-shaped.

According to an alternative aspect of the present disclosure the cutouts on the first side have a truncated V-shape.

For increasing the strength of the catheter, all angles in the cutouts have a radius according to a further aspect of the present disclosure.

According to yet another aspect of the present disclosure the distance between the center of the cutouts on the first side is equal to the distance between the cutouts on the second side.

According to yet an alternative aspect of the present disclosure the cutouts on the first side are opposite the cutouts on the second side.

According to an advantageous aspect of the present disclosure at least one guard wire is arranged on the catheter to hold the pull wire adjacent the flexible tube. The guard wire prevents the pull wire to deviate from the bend in the distal part of the catheter.

According to another aspect of the present disclosure the guard wire is arranged circumferentially around the flexible tube.

According to yet another aspect of the present disclosure one end of the guard wire is attached to the distal end of the flexible tube, the other end of the guard wire is attached to the proximal end of the catheter, and wherein the guard wire is arranged in a coil around the flexible tube between the distal end of the flexible tube and the proximal end of the catheter.

According to yet an aspect of the present disclosure a flexible sheath is arranged on the flexible tube enclosing the pull wire.

According to yet a further aspect of the present disclosure the outer diameter of the hollow tube is larger than 1,3 mm and smaller than 2,1 mm. This interval covers catheter diameters from 4 French to 6 French (1 French = 1/3 mm). The length of the catheter is preferably at least 100 cm and up to 130 cm, more preferable between 110 cm and 120 cm.

According a further aspect of the present disclosure the distal end of the flexible tube is equipped with a tracking device. The tracking device could for instance be an RF coil or specific material detectable by MRI (magnetic resource imaging). Further, the entire shape and orientation of the catheter's flexible tube is preferably also detectable. Thus, according to one further aspect the flexible tube is equipped with a tracking function, such as RF coils or coatings including for instance a contrast agent such as gadolinium or iodine.

A further object of the present disclosure is to provide a handle that facilitates using unilateral catheters.

Thus, according to one aspect of the present disclosure a catheter handle is provided having a distal end and a proximal end. The handle comprises an elongate housing extending between the proximal end and the distal end, wherein the housing has an opening at the distal end arranged to allow one pull wire for a unidirectional catheter to run through, wherein a holding device is slidably arranged along and inside the housing and arranged to be fixed to the pull wire. The handle further comprises a pull wire control arranged such that it is accessible on the outside of the housing and connected through a side opening in the housing to the holding device, wherein the pull wire control is movable between a distal position such that when in use and connected to a pull wire, the pull wire connected to the holding device is in a relaxed state and a proximal position in which the pull wire is in a tense state such that the unidirectional catheter is in a bent state, wherein the pull wire control is arranged circumferentially around the distal end of the housing.

According to a further aspect of the present disclosure the pull wire control is turnably arranged such that it can be turned between a released state and a locked state, wherein in the released state the pull wire control is arranged such that it is movable between the distal position and the proximal position along the housing to straighten or bend the catheter, respectively, when connected to a pull wire.

According to yet a further aspect of the present disclosure the holding device is turnably arranged between a released state and a locked state together with the pull wire control, the holding device having at least one periphery extension that is arranged in one of several recesses arranged on the inside of the housing, when in a locked state.

The handle comprises according to another aspect of the present disclosure a spring, wherein one end of the spring is arranged in the housing and the other end is arranged on the holding device such that the holding device is biased towards a locked state.

For improved stabilization of the pull wire control a directing tube is according to yet an aspect of the present disclosure arranged at the distal opening of and in line with the housing and such that pull wire control is slidably arranged at its distal end circumferentially around the directing tube.

According to another aspect of the present disclosure a method for navigating a unilateral catheter to an endocardial site, the lungs, the epicardial site via the coronary vessels, or abdominal organs, is provided. The method is possible to use to reach the lungs or abdominal organs. The method comprises: advancing the catheter through an opening punctured in a radial access opening, a femoral artery, a vein, or in a cubital vein towards the desired site, when reaching a curve or bifurcation in a blood vessel with a distal end of the catheter, pulling a pull wire of the catheter until a bend of a distal end of the catheter is detectable, rotating the catheter by rotating a handle of the catheter for aligning the radial orientation of the bend of the distal end of the catheter to correspond to a radial orientation of the curve or bifurcation in the blood vessel, pulling the pull wire by manually operating the handle of the catheter until the radius of the bend in the distal end of the catheter corresponds to a radius of the curve or bifurcation in the blood vessel, and pushing the handle for advancing the catheter through the curve or bifurcation.

According to one aspect of the method it further comprises releasing the pull wire to straighten out the distal end of the catheter.

Preferably, the method comprises using a catheter according to above disclosure.

Also preferably, the method comprises using a handle according to the above disclosure.

According to an aspect of the present disclosure a medical method is provided of accessing a site in or at a heart of a patient through the vasculature of the patient from a radial or cubital vessel, including the method discloses above for navigating to said heart.

It should be emphasized that the term "comprises/comprising" when used in this specification is taken to specify the presence of stated features, integers, steps or components but does not preclude the presence or addition of one or more other features, integers, steps, components or groups thereof.

Further features of, and advantages with, the present invention will become apparent when studying the appended claims and the following description. The skilled person realize that different features of the present invention may be combined to create embodiments other than those described in the following, without departing from the scope of the present invention.

### BRIEF DESCRIPTION OF THE DRAWINGS

The above, as well as additional objects, features and advantages of the present invention, will be better understood through the following illustrative and non-limiting detailed description of exemplary embodiments of the present invention, wherein:
Figure 1 is a perspective view of an embodiment of the catheter according to the present disclosure,
Figure 2 is the perspective view of the embodiment of the catheter in figure 1 without a flexible sheath,
Figure 3 is an enlarged part of the distal end of the catheter in figure 2,
Figure 4 is a sideview of the distal end of the catheter in figure 3,
Figure 5 is an exploded view of the distal part of the catheter in figure 3,
Figure 6 is a sideview of a part of the flexible tube in the catheter according to an embodiment of the present disclosure,
Figure 7 is an enlarged view of a part of the flexible tube in figure 6,
Figure 8 is a perspective of a handle according to an embodiment of the present disclosure,
Figure 9 is a partial cross section of the handle in figure 8,
Figure 10 is an enlarged view of a part in figure 9,
Figure 11 is a partial cross section of a handle according to an embodiment of the present disclosure,
Figure 12 is a side view of a distal end of the catheter in bent state, and
Figure 13 is a perspective view of a catheter with dilution apertures.

### DETAILED DESCRIPTION OF EXAMPLE EMBODIMENTS OF THE INVENTION

The present invention will now be described more fully hereinafter with reference to the accompanying drawings, in which exemplary embodiments of the invention are shown. The invention may, however, be embodied in many different forms and should not be construed as limited to the embodiments set forth herein; rather, these embodiments are provided for thoroughness and completeness. Like reference character refer to like elements throughout the description.

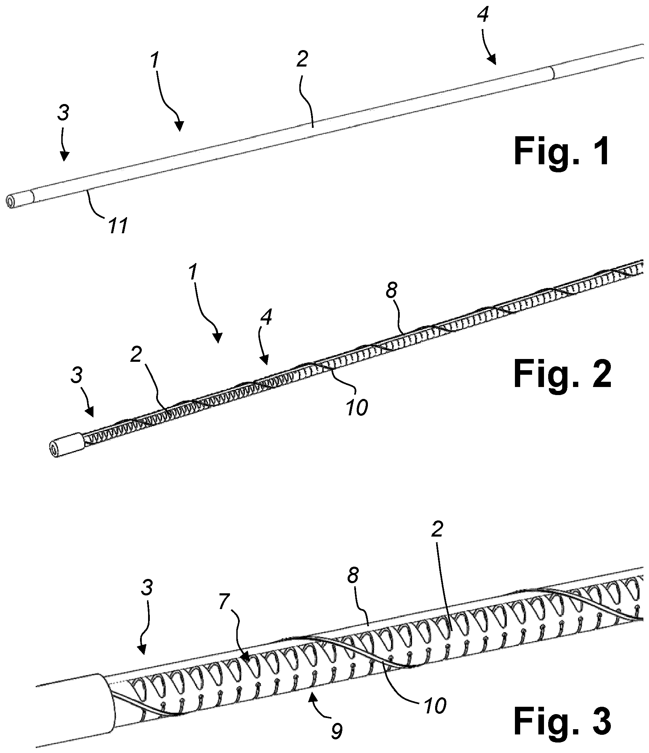

With reference to figures 1 and 2, a catheter 1 is shown. Figure 1 shows a flexible sheath 11 that is arranged on a flexible tube and enclosing a pull wire 8.

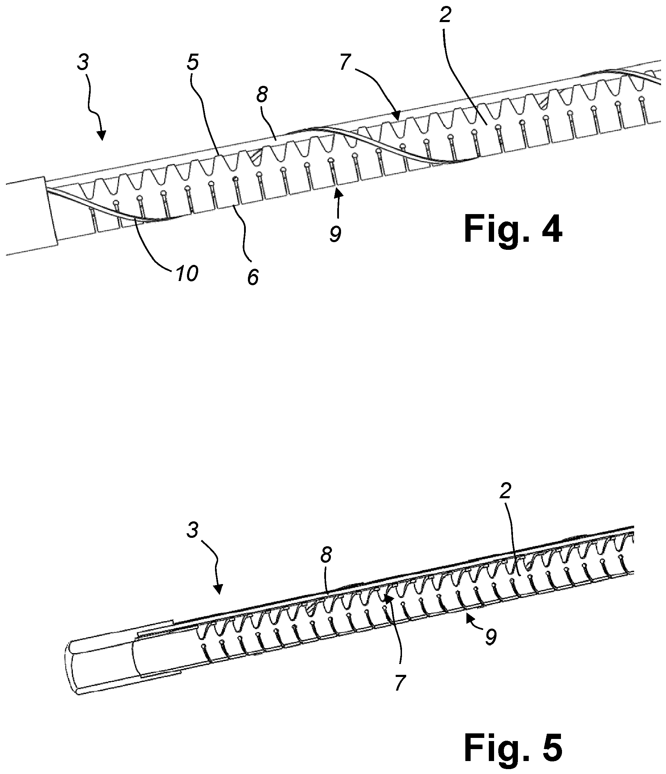

Looking at figures 3, 4 and 5, a catheter 1 comprises a flexible and cylindrical hollow tube 2 at a distal end, wherein the flexible tube 2 has two opposite ends 3, 4, a first side 5 and a to the first side 5 opposite second side 6 extending between the two opposite ends 3, 4. The first side 5 has cutouts 7 along at least a part of its length, and wherein a pull wire 8 is arranged at the most distal end 3 and on the first side 5 of the flexible tube 2. Further, the second side 6 has cutouts 9 along a corresponding length to the first side 5 and wherein the cutouts 7 on the first side are wider than the cutouts 9 on the second side 6.

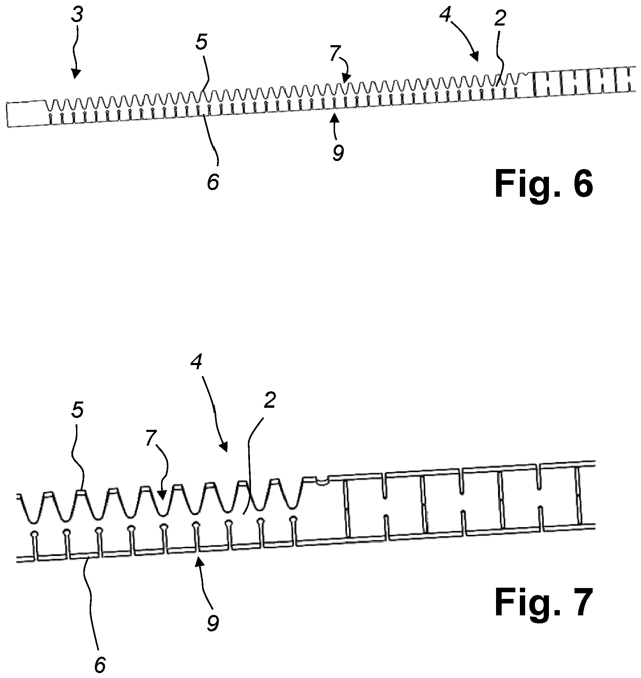

The cutouts 7 on the first side 5 are V-shaped and have to an extent truncated V-shape. As further can be seen, all angles in the cutouts have a radius for improving strength in the hollow flexible tube to withstand repeated bending, as can be seen more clearly in figures 6 and 7.

The distance between the center of the cutouts 7 on the first side 5 is equal to the distance between the cutouts 9 on the second side 6 and the cutouts 7 on the first side 5 are also arranged opposite the cutouts 9 on the second side 6.

One guard wire 10 is arranged on the catheter 1 to hold the pull wire 8 adjacent the flexible tube. The guard wire 10 is arranged circumferentially around the flexible tube 2.

Further, one end of the guard wire 10 is attached to the distal end 3 of the flexible tube, the other end of the guard wire 10 is attached to the proximal end of the catheter, and the guard wire 10 is arranged in a coil around the flexible tube 2 between the distal end 3 of the flexible tube 2 and the proximal end of the catheter 1.

The guard wire 10 protects the flexible sheath 11 when the flexible tube 2 is bent by pulling the pull wire 8 and prevents the pull wire 8 to cut through the flexible sheath and thus provide for better strength without increasing the dimensions of the catheter.

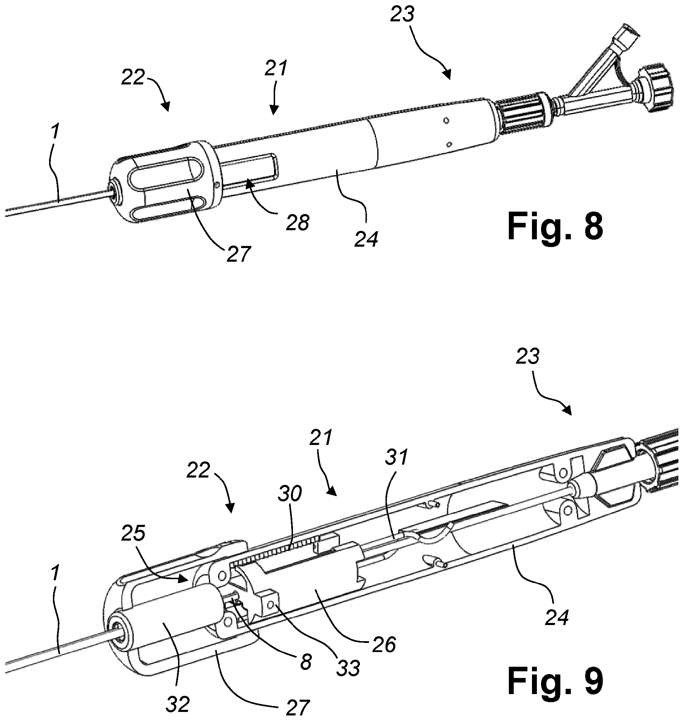

Turning to figure 8, an embodiment of a handle 21 is shown. As is visible in figure 9, a catheter handle 21 has a distal end 22 and a proximal end 23. The handle 21 comprises an elongate housing 24 extending between the proximal end 23 and the distal end 22. The housing 24 has an opening 25 at the distal end 22 arranged to allow one pull wire 8 for a unidirectional catheter 1 to run through. A holding device 26 is slidably arranged along and inside the housing 24 and arranged to be fixed to the pull wire 8. The handle 21 further comprises a pull wire control 27 arranged such that it is accessible on the outside of the housing 24 and connected through a side opening 28, see figure 1, in the housing 24 to the holding device 26. The pull wire control 27 is movable between a distal position such that when in use and connected to a pull wire 8, the pull wire 8 connected to the holding device 26 is in a relaxed state, as in figure 9, and a proximal position in which the pull wire 8 is in a tense state such that the unidirectional catheter 1 is in a bent state. The pull wire control 27 is arranged circumferentially around the distal end 22 of the housing 24.

Also, the pull wire control 27 is turnably arranged such that it can be turned between a released state and a locked state. In the released state the pull wire control 27 is arranged such that it is movable between the distal position and the proximal position along the housing 24 to straighten or bend the catheter 1, respectively.

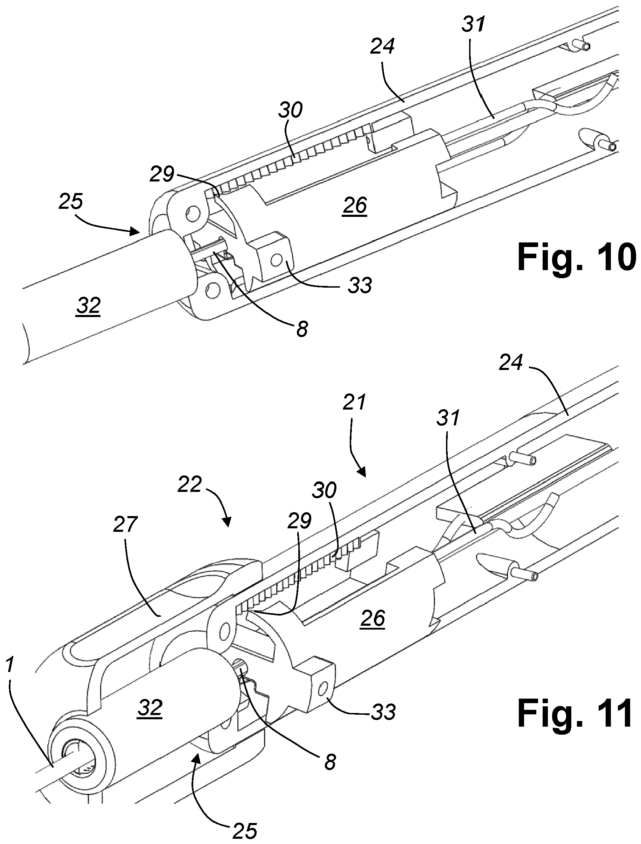

As is further indicated in figures 9 to 11 the holding device 26 is turnably arranged between a released state and a locked state together with the pull wire control 27, the holding device 26 having one periphery extension 29 that is possible to arrange in one of several recesses 30 arranged on the inside of the housing 24.

Further, the handle 21 comprises a spring 31, wherein one end of the spring 31 is arranged in the housing 24 and the other end is arranged on the holding device 26 such that the holding device 26 is biased towards a locked state.

A directing tube 32 is arranged at the distal opening 25 of and in line with the housing 24 and that pull wire control 27 is slidably arranged at its distal end 22 circumferentially around the directing tube 32.

Also shown in figures 9 to 11 is a protrusion 33 on the holding device 26 for connecting with a corresponding recess (not shown) on the inside of the pull wire control 27 and to thereby establish the connection between the pull wire control 27 and the holding device 26.

The method for navigating a unilateral catheter, as shown in figures 1 to 7, to a desired site, for instance, an endocardial site, the lungs, or abdominal organs, comprises: advancing the catheter 1 through an opening punctured in a radial access opening or in a cubital vein towards the desired site, when reaching a curve or bifurcation in a blood vessel with a distal end 3 of the catheter 1, pulling a pull wire 8 of the catheter 1 until a bend of a distal end 3 of the catheter 1 is detectable, rotating the catheter 1 by rotating a handle 21, as shown in figures 8 to 11, of the catheter 1 for aligning the radial orientation of the bend of the distal end 3 of the catheter 1 to correspond to a radial orientation of the curve or bifurcation in the blood vessel, pulling the pull wire 8 by manually operating the handle 21 of the catheter 1 until the radius of the bend in the distal end 3 of the catheter 1 corresponds to a radius of the curve or bifurcation in the blood vessel, and pushing the handle 21 for advancing the catheter 1 through the curve or bifurcation.

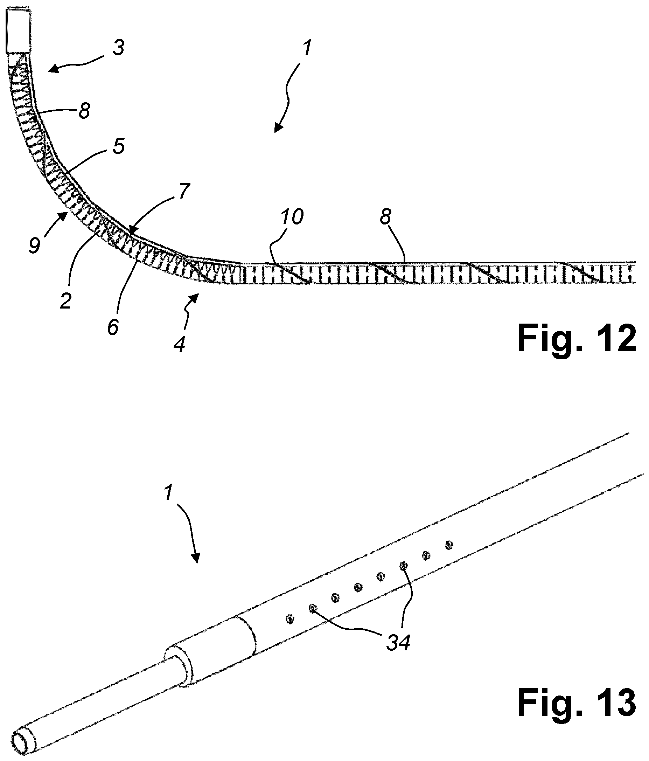

Figure 12 shows the catheter 1 when the pull wire 8 is "pulled", i.e. the distal section of the catheter or flexible tube 2 is bent. The cutouts 7 on the first side 5 are narrower in the bent state of the catheter 1 whereas the cutouts 9 on the second side 6 are slightly wider.

When the pull wire 8 is pulled the flexible tube 2 will bend with the pull wire 8 on the inside of the bend since it is placed on the first side 5 of the flexible tube 2 where the cutouts 7 are larger compared to the cutouts 9 on the second side 6. Thus, the flexible tube is biased to bend in the direction on the side where the pull wire 8 is arranged. The catheter is covered with a flexible sheath (11 in figure 1) and in order for this sheath not to be ripped or cut by the pull wire 8, a guard wire 10 is arranged in a coil round the flexible tube 2 such that the guard wire 10 keeps the pull wire 8 close to the flexible tube 2 when the catheter is in a bent state. By having the guard wire, the thickness of the flexible sheath can be kept to a minimum and thus allowing for the small dimensions of the catheter 1.

After the curve or bifurcation, the pull wire 8 is released to straighten out the distal end 3 of the catheter 1.

Also, a further function of the present catheter 1 is to have dilution apertures 34 on the side at the distal end of the catheter in order to be able to release contrast agents. Figure 13 shows an example with such apertures 34. Preferably, the apertures 34 should not be located on the outer curve of the deflectable part of the catheter 1 to avoid protrusion of small devices that are being fed through the catheter.

The medical method of the present disclosure of accessing a site in or at a heart of a patient through the vasculature of the patient from a radial or cubital vessel, includes the above-described method for navigating to said heart.

As used herein, the singular forms "a", "an" and "the" are intended to include the plural forms as well, unless expressly stated otherwise. It will be further understood that the terms "includes," "comprises," "including" and/or "comprising," when used in this specification, specify the presence of stated features, integers, steps, operations, elements, and/or components, but do not preclude the presence or addition of one or more other features, integers, steps, operations, elements, components, and/or groups thereof. It will be understood that when an element is referred to as being "connected" or "coupled" to another element, it can be directly connected or coupled to the other element or intervening elements may be present.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

While several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. A catheter (1) for delivery and sampling comprises a flexible and cylindrical hollow tube (2) at a distal end, the flexible tube (2) has two opposite ends (3, 4), a first side (5) and a to the first side (5) opposite second side (6) extending between the two opposite ends (3, 4), wherein the first side (5) has cutouts (7) along at least a part of its length, and wherein a pull wire (8) is arranged at the most distal end (3) and on the first side (5) of the flexible tube (2),
**characterized in**
**that** the second side (6) has cutouts (9) along a corresponding length to the first side (5) and wherein the cutouts (7) on the first side are wider than the cutouts (9) on the second side (6).

2. The catheter (1) according to claim 1, wherein the cutouts (7) on the first side are V-shaped.

3. The catheter (1) according to claim 1, wherein the cutouts (7) on the first side have a truncated V-shape.

4. The catheter (1) according to any of the preceding claims, wherein all angles in the cutouts have a radius.

5. The catheter (1) according to any of the preceding claims, wherein the distance between the center of the cutouts (7) on the first side (5) is equal to the distance between the cutouts (9) on the second side (6).

6. The catheter (1) according to any of the preceding claims, wherein the cutouts (7) on the first side (5) are opposite the cutouts (9) on the second side (6).

7. The catheter (1) according to any of the preceding claims, wherein at least one guard wire (10) is arranged on the catheter (1) to hold the pull wire (8) adjacent the flexible tube.

8. The catheter (1) according to claim 7, wherein the guard wire (10) is arranged circumferentially around the flexible tube (2).

9. The catheter (1) according to claim 7, wherein one end of the guard wire (10) is attached to the distal end (3) of the flexible tube, the other end of the guard wire (10) is attached to the proximal end of the catheter, and wherein the guard wire (10) is arranged in a coil around the flexible tube (2) between the distal end (3) of the flexible tube (2) and the proximal end of the catheter (1).

10. The catheter (1) according to any of the preceding claims, wherein the outer diameter of the hollow tube (2) is larger than 1,3 mm and smaller than 2,1 mm.

11. The catheter (1) according to any of the preceding claims, comprising a catheter handle (21) having a distal end (22) and a proximal end (23), the handle (21) comprising an elongate housing (24) extending between the proximal end (23) and the distal end (22), wherein the housing (24) has an opening (25) at the distal end (22) arranged to allow one pull wire (8) for a unidirectional catheter (1) to run through, wherein a holding device (26) is slidably arranged along and inside the housing (24) and arranged to be fixed to the pull wire (8), wherein the handle (21) further comprises a pull wire control (27) arranged such that it is accessible on the outside of the housing (24) and connected through a side opening (28) in the housing (24) to the holding device (26), wherein the pull wire control (27) is movable between a distal position such that when in use and connected to a pull wire (8), the pull wire (8) connected to the holding device (26) is in a relaxed state and a proximal position in which the pull wire (8) is in a tense state such that the unidirectional catheter (1) is in a bent state, wherein the pull wire control (27) is arranged circumferentially around the distal end (22) of the housing (24).

12. The catheter (1) according to claim 11, wherein the pull wire control (27) is turnably arranged such that it can be turned between a released state and a locked state, wherein in the released state the pull wire control (27) is arranged such that it is movable between the distal position and the proximal position along the housing (24) to straighten or bend the catheter (1), respectively, when connected to a pull wire (8).

13. The catheter (1) according to any of the claims 11 and 12, wherein the holding device (26) is turnably arranged between a released state and a locked state together with the pull wire control (27), the holding device (26) having at least one periphery extension (29) that is arranged in one of several recesses (30) arranged on the inside of the housing (24), when in a locked state.

14. The catheter (1) according to claim 13, wherein the handle (21) comprises a spring (31), wherein one end of the spring (31) is arranged in the housing (24) and the other end is arranged on the holding device (26) such that the holding device (26) is biased towards a locked state.

15. The catheter (1) according to any of the claims 11 to 14, wherein a directing tube (32) is arranged at the distal opening (25) of and in line with the housing (24) and that pull wire control (27) is slidably arranged at its distal end (22) circumferentially around the directing tube (32).
